# NEW EUROPEAN PATENT SPECIFICATION

(11) **EP 0 637 446 B2**
(45) Date of publication and mention of the opposition decision: **22.06.2011**
(45) Mention of the grant of the patent: 22.08.2001
(21) Application number: 94304078.2
(22) Date of filing: 07.06.1994
(51) Int. Cl.: A61C 15/04

(54) **Dental floss provided with chemotherapy agents**
Zahnseide mit chemotherapeutischen Wirkstoffen
Fil dentaire avec agents chimiotherapiques

(30) Priority: 08.06.1993 BR 9301968
(43) Date of publication of application: 08.02.1995
(73) Proprietor: JOHNSON & JOHNSON CONSUMER PRODUCTS, INC., Skillman, New Jersey 08858-9418 (US)
(72) Inventor: Simionato, Luiz, Rua Rua Esperanca, No. 227, Sao Jose Dos Campos, Sao Paulo (BR); Netto, Emilson I, Variante Getulio Vargas, No.1919, Jacarei, Sao Paulo (BR); Porsani, Dorival F., ... (BR)
(74) Representative: Mercer, Christopher Paul

(56) References cited:
- WO-A1-91/14412
- WO-A1-92/10978
- WO-A1-93/02633
- US-A- 2 667 443
- US-A- 2 772 205
- US-A- 3 838 702
- US-A- 3 943 949
- US-A- 5 098 711
- US-A- 5 165 913
- DATABASE WPI Week 198948, Derwent Publications Ltd., London, GB; AN 89-352083, XP002987273 & JP 1 262 857 A (DENTAL KAGAKU KK) 19 October 1989

## Description

This invention relates to dental waxed textile materials (threads and tapes) that contain chemotherapy agents.

In order to facilitate understanding of this specification, any reference to dental floss should be understood to extend to dental tape, dental thread or any similar materials.

### STATE OF THE ART

Various patent documents that illustrate the state of the art relating to this invention are mentioned below:

US Patent 4,548,219 and US Patent 4,638,823 disclose a dental floss containing a stannous fluoride incorporated in the floss by means of a soluble polyethylene glycol wax.

Patent application WO9108716 describes a dental floss in the absence of bonding agents and including a surfactant, a specific silicone type coating and a chemotherapy agent.

Patent application EP 335466 discloses a low friction coefficient polytetrafluoroethylene dental floss containing active agents.

US Patent 3838702 discloses a dental floss provided with a coating of polymer, elastomer, natural or synthetic wax, which contains inorganic particles that act as a polishing agent for the teeth.

US Patent 4029113 mentions a dental floss (and its manufacturing process) provided with natural or synthetic wax that releases fluorine ion during use. Its manufacturing process comprises passing an elongated unwaxed dental textile material through an aqueous emulsion of wax containing the fluorine-containing compound.

US Patent 5040554 mentions a dental floss with a wax, soap or detergent bonding agent, as a base for a phenol derivative germicide coating.

US Patent 2667443 relates to dental floss impregnated with chemicals for the purpose of increasing the resistance of teeth to dental caries. The dental floss can contain a basic compound with or without a binder such as beeswax, a petroleum wax, a resin, resinous wax-like material, or a water soluble wax-like material (e.g. solid ethylene glycol polymer or derivatives).

US Patent 2772205 relates to a dental floss containing a higher aliphatic acyl amide of an amino carboxylic acid compound to inhibit acid production in the mouth for a prolonged period of time. To render the floss smooth a wax-like material such as beeswax, paraffin, petroleum wax, solid polyethylene glycols, wax-like resins and polymers, microcrystalline waxes can be applied to the floss.

JP Patent application 12-62857 discloses a dental floss containing a defined inorganic calcium particulate material and a specified wax.

US Patent 3943949 discloses stabilised, flavoured dental floss comprising filaments with a wax coating containing spray dried flavour particles.

US Patent 4414990 discloses a dental floss comprising filaments coated with a wax coating that is in turn coated with a polymeric coating containing a fluoride salt.

WO 91/14412 discloses a method of adding a chemotherapeutic preparation to a multifiber dental floss wherein the preparation contains a surfactant to clean debris and plaque during flossing and a coating substance to form a fugitive, thin transparent coating on oral cavity surfaces.

It should be noted that waxed dental flosses containing active agents are well known in the present state of the art, both for the mechanical removal of food deposits, plates or tartar from the internal spaces between teeth, and for the release of active agents that will act within the oral environment, such as tooth decay prevention, anti-tartar, antibiotic, antimicrobial, anti-inflammatory, antioxidant, antiseptic, anticoagulant, analgesic, anesthetic, desensitization, and flavoring products, among others.

The use of wax (herein construed as actual wax or waxy material of any kind) for impregnation of dental floss is already known, basically as a bonding agent, that is to say, to maintain the threads together, which make up the dental floss. Furthermore the wax acts as a lubricant that provides more comfort when using the dental floss, promoting sliding and penetration of floss between teeth.

During the development of the technology related to the subject matter hereof, wax began to be used as a support for active agents, mainly for products with a fluorine content.

It was found that the release of the active agents added to the wax was of inefficient, due to the insoluble nature of waxes used up to then. Soluble waxes then began to be used, such as polyethylene glycol derivative waxes.

It was then found that, in spite of the efficiency in the release of the active agents contained in dental floss having soluble waxes, use became less comfortable, that is to say, with harsher sliding and much harder penetration between teeth, and thus less comfortable for the user.

Such effects were accounted for by a possible increase in the coefficient of sliding friction between dental floss and teeth, partially solved by using more noble material, such as polytetrafluoroethylene. However, this entailed a much higher cost for the floss, both as a result of the intrinsic cost of raw material itself, and of the manufacturing procedure.

Therefore the need still remains for an improved dental floss that would entirely or partially solve the problems found to date.

An object of the present invention is to provide a dental floss which has improved chemotherapy product release qualities within the oral environment, as well as comfort in use sliding and penetration between teeth.

Still another object of the present invention is to provide an improved dental floss with improved performance combined with low cost.

According to the present invention there is provided dental floss as defined in claim 1.

This present invention aims at eliminating or mitigating the problems found by the present state of the art products, by means of a dental floss provided with an emulsible wax.

Unexpectably, it has been found that such a wax allows for an efficient release of any associated chemotherapy product, while concurrently preserving the comfortable effect assured by dental flosses with insoluble wax, that means, the same sliding and penetration between teeth.

In the dental floss of the present invention, the wax is the carrier for the active agents within the oral cavity.

The dental floss of this invention is made up of known filamentary materials, such as cotton, silk, nylon, polyethylene, polypropylene, polyester, acrylic fibers, any combination thereof, etc., having characteristics that are also known, such as the number of filaments per thread, the amount of twisting applied, tensile strength, etc. These are known parameters for person skilled in the art and do not impose any restrictions on the scope of the invention.
Ethoxylated sorbitan monostearate products such as Nikkol TS by Nikko Corporation, Japan are suitable;
Glyceryl monostearate products such as Atmul by Imperial Chemical Industries, England, or Imwitor, by Huels America, USA are suitable.
Ethoxylated glyceryl monostearate products such as Cutina E-24 of Henkel, Germany, or Varonic L142 by Sherex, USA are suitable.

Suitably the chemotherapy agent includes at least one product with at lease one of the following characteristics: tooth decay prevention, antitartar, antibiotic, antimicrobial, anti-inflammatory, antioxidant, antiseptic, anticoagulant, analgesic, anesthetic and desensitizing.

Preferably the floss of this invention contains a chemotherapy agent that has tooth decay characteristics, such as sodium fluoride and/or stannous fluoride as the active agent in the oral cavity, without excluding any others known to persons skilled in the art.

Impregnation with wax of the thread material of the dental floss is made according to a process known in the art, for instance, by dipping the floss into a bath of molten emulsible wax, or any other.

An example of a practical embodiment of the invention is given below merely by way of illustration, without this imposing any restriction or limit on the scope of the invention, as defined in the claims.

Two dental flosses A and B are manufactured pursuant to the process described by US Patent 4,029,113, with threads (12 nylon yarns of 78 Dtex per yarn, 23 filaments per yarn and 120 twists/m) impregnated with 20% by weight of, respectively, emulsible wax according to invention (LIPOWAX JDL, lonquimica, Brazil) and polyethylene glycol (CARBOWAX 1450, Union Carbide, United States). Both the emulsible wax and the polyethylene glycol contain a sufficient quantity of sodium fluoride so that an approximate amount of 300 ppm of this matter is obtained in the resulting floss.

Subsequent tests showed that:
- both floss A and floss B permit release of equivalent quantities of fluorine within the oral cavity during use;
- floss A of the invention is clearly more comfortable to use, that is to say, it has better sliding and penetration properties than floss B which is common to the present state of the art.

## Claims

1. Dental floss provided with at least one chemotherapy agent, wherein said floss further includes a wax that is emulsible upon contact with saliva, the wax including at a least one surfactant capable of emulsifying said wax upon contact with saliva, **characterized in that** said emulsible wax includes:
25% to 40% by weight of refined beeswax;
4% to 10% by weight of microcrystalline wax;
23% to 35% by weight of ethoxylated sorbitan monostearate;
3% to 10% by weight of glyceryl monostearate; and
22% to 38% of ethoxylated glyceryl monostearate.

2. Dental floss according to Claim 1, **characterized in that** said at least one chemotherapy agent includes at least one product with at least one of the following characteristics: tooth decay prevention, antitartar, antibiotic, antimicrobial, anti-inflammatory, antioxidant, antiseptic, anticoagulant, analgesic, anesthetic and desensitizing.

3. Dental floss according to Claim 1 or Claim 2, **characterized in that** said at least one chemotherapy agent has tooth decay prevention characteristics.

4. Dental floss according to Claim 3, **characterized in that** said chemotherapy agent is selected from sodium fluoride and stannous fluoride.

## Patentansprüche

1. Zahnseide mit wenigstens einem chemotherapeutischen Wirkstoff versehen, wobei die Zahnseide weiterhin ein Wachs enthält, das bei Kontakt mit Speichel emulgierbar ist, wobei das Wachs mindestens einen oberflächenaktiven Stoff enthält, der in der Lage ist, das Wachs bei Kontakt mit Speichel zu emulgieren, **dadurch gekennzeichnet, dass** das emulgierbare Wachs enthält:
25% bis 40% (Gew.-%) raffiniertes Bienenwachs;
4% bis 10% (Gew.-%) mikrokristallines Wachs;
23% bis 35% (Gew.-%) ethoxyliertes Sorbitanmonostearat;
3% bis 10% (Gew.-%) Glycerylmonostearat; und
22% bis 38% ethoxyliertes Glycerylmonostearat.

2. Zahnseide nach Anspruch 1, **dadurch gekennzeichnet, dass** der mindestens eine chemotherapeutische Wirkstoff mindestens ein Produkt mit mindestens einem der folgenden Kennzeichen enthält: vorbeugend gegenüber Zahnverfall, Zahnsteinbildung verhindernd, antibiotisch, antimikrobiell, entzündungshemmend, antioxidierend, antiseptisch, antikoagulierend, analgetisch, anästhetisierend und desensibilisierend.

3. Zahnseide nach Anspruch 1 oder Anspruch 2, **dadurch gekennzeichnet, dass** der mindestens eine chemotherapeutische Wirkstoff vorbeugende Eigenschaften gegenüber Zahnverfall aufweist.

4. Zahnseide nach Anspruch 3, **dadurch gekennzeichnet, dass** der chemotherapeutische Wirkstoff aus Natriumfluorid und Zinn(II)-fluorid ausgewählt ist.

## Revendications

1. Fil dentaire pourvu d'au moins un agent de chimiothérapie, **caractérisé en ce que** ledit fil contient aussi une cire émulsionnable au contact de la salive, la cire contenant au moins un tensioactif capable d'émulsionner ladite cire au contact de la salive, **caractérisé en ce que** ladite cire émulsionnable contient :
25% à 40% en poids de cire d'abeille raffinée ;
4% à 10% en poids de cire microcristalline ;
23% à 35% en poids de monostéarate de sorbitane éthoxylé ;
3% à 10% en poids de monostéarate de glycéryle ; et
22% à 38% de monostéarate de glycéryle éthoxylé.

2. Fil dentaire selon la revendication 1, **caractérisé en ce que** ledit agent de chimiothérapie, au nombre d'au moins un, contient au moins un produit possédant au moins une des caractéristiques suivantes : prévention des caries dentaires, antitartre, antibiotique, antimicrobien, anti-inflammatoire, antioxydant, antiseptique, anticoagulant, analgésique, anesthésique et désensibilisant.

3. Fil dentaire selon la revendication 1 ou la revendication 2, **caractérisé en ce que** ledit agent de chimiothérapie, au nombre d'au moins un, a des caractéristiques de prévention des caries.

4. Fil dentaire selon la revendication 3, **caractérisé en ce que** ledit agent de chimiothérapie est choisi parmi le fluorure de sodium et le fluorure d'étain.
